# EUROPEAN PATENT APPLICATION

(11) **EP 3 150 257 A1**
(43) Date of publication of application: **05.04.2017**
(21) Application number: 15187707.3
(22) Date of filing: 30.09.2015
(51) Int. Cl.: A61P 37/04, A61K 33/42, A61K 38/43, A61K 45/06

(54) **A METHOD FOR PRODUCING A COMPOSITION FOR STRENGTHENING THE IMMUNE SYSTEM**

(71) Applicant: Buga, Doris, 83178 Puchheim (DE)
(72) Inventor: Buga,, Roman, 82178 Puchheim (DE)
(74) Representative: Engelhard, Markus

(57) **Abstract**

The present invention relates to a method for producing a composition for strengthening the immune system. The present invention also relates to composition produced by such method as well as to uses of such composition. The composition in accordance with the present invention may be used to increase the blood electric charge in patients. It is particularly useful in a method of preventing, treating and/or alleviating a disease selected from infections, in particular viral and bacterial infections, cancer, diabetes and/or depression in a patient. It can also be used in a method of mood-enhancing a patient.

## Description

The present invention relates to a method for producing a composition for strengthening the immune system. The present invention also relates to a composition produced by such method as well as to uses of such composition.

A number of drugs are available that are used to increase the immune status of a patient and to fight infections. For example, extracts from Echinacea purpurea have been used to treat viral infections and/or to strengthen the immune system. The preparation of such extracts requires an elaborate and relatively complicated procedure. Likewise, an extract from Pelagonium sidoides is used to treat viral and bacterial infections. It is, however, not clear whether this is really effective, and the extraction procedure, again, is relatively elaborate.

Accordingly, there exists a need in the art for compositions for strengthening the immune system and for methods providing such compositions.

It was therefore an object of the present invention to provide for means to strengthen the immune system of a patient which means are easy and straightforward to provide and which are economically affordable.

In a first aspect, the present invention relates to a method for producing a composition for strengthening the immune system, said method comprising:
a) providing in any order,
   - a protein source,
   - a phosphate source,
   - at least one enzyme,
      and mixing them to form a mixture;
b) introducing mechanical energy into the mixture while keeping the mixture at a temperature in the range of from -20°C to 42°C;
c) drying the mixture at a temperature in the range of from -100°C to 380°C and comminuting the mixture to granules to obtain a composition for strengthening the immune system, said composition consisting of said granules of said mixture. In a preferred embodiment, during step b), no water or ice is added.

In other preferred embodiments e. g. when the protein source is milk powder, water may be added at an amount of up to 20 % (weight/weight) of the mixture.

Drying can be performed by any means suitable and known to a person skilled in the art. For example, drying may be achieved by exposing the mixture to an atmosphere at elevated temperature(s), for example in an oven or by keeping the mixture in the vicinity of a heating element, such as a radiant heater. In another embodiment, the drying in step c) may be achieved by performing a freeze-drying procedure. Typically, in such freeze-drying procedure, the mixture is frozen to a low temperature such as -100°C or even further below, for example -200°C - -196°C, and thereafter the surrounding pressure around the mixture is reduced to allow the water contained in the mixture that had been frozen, to sublimate directly from the solid phase into the gas phase. The freezing of the mixture to such low temperatures may be achieved by any means suitable in the art, for example introducing the mixture into liquid nitrogen and allowing it to freeze therein.

In one embodiment, during step b), the introducing of mechanical energy into the mixture leads to a temperature increase of said mixture, and the introducing of mechanical energy into said mixture in step b) is performed such that said temperature increase of said mixture is in a range of from 3°C to 62°C. Without wishing to be bound by any theory, the inventor believes that the more mechanical work is introduced and the higher the temperature risk, the higher the energy of the resultant composition becomes.

In one embodiment, step b) is performed by a process selected from a cutting, mincing, shaking, kneading, mixing, tumbling, rolling, pressing, beating, pushing, squeezing, rubbing, and combinations of any of the foregoing.

In one embodiment, the drying in step c) is performed until the mixture has a water activity (a_{w}) in the range of from 0.01 to 0.99, preferably from 0.3 to 0.9, more preferably from 0.5 to 0.8.

In one embodiment, the drying in step c) is performed until the mixture has a water activity in the range of from 0.5 to 0.7, preferably 0.5 to 0.6. In another embodiment, the drying in step c) is performed until the mixture has a water activity in the range of from 0.01 to 0.3, preferably 0.01 to 0.1. These particularly low values of water activity may be achieved, in particular if one uses very intense drying processes, such as freeze drying.

In one embodiment, the comminuting in step c) is performed until the granules of said mixture have an average diameter in the range of from 1 µm to 10 mm, preferably 10 µm to 1 cm, more preferably 100 µm to 1 cm. Again, without wishing to be bound by any theory, the inventor believes that the smaller the size of the granules, the higher the energy of the composition becomes.

In one embodiment, the protein source is selected from meat, bones, skin, cartilage, offals, from mammals, such as cow, pig, sheep, goat, camel, horse, in particular beef, pork, lamb; birds, in particular poultry, such as chicken, duck, ostrich, goose; fish and seafood, such as lobster, shrimp, crab, crayfish; and animal products, such as milk and eggs; vegetables, in particular beans, nuts, peas, peanuts, corn, cabbage, legumes, cereals, seeds, kernels and parts and products thereof, e. g. tofu;
and/or wherein the phosphate source is selected from monosodium phosphate (NaH₂PO₄), disodium phosphate (Na₂HPO₄), trisodium phosphate (Na₃PO₄), ortho-phosphoric acid (H₃PO₄), monopotassium phosphate (KH₂PO₄), dipotassium phosphate (K₂HPO₄), tripotassium phosphate (K₃PO₄), calcium phosphate, in particular monocalcium phosphate (Ca(H₂PO₄)₂, dicalcium phosphate (CaHPO₄), tricalcium
phosphate Ca₃(PO₄)₂, magnesium phosphate, in particular monomagnesium phosphate (Mg(H₂PO₄)₂), dimagnesium phosphate (MgHPO₄), trimagnesium phosphate (Mg₃(PO₄)₂), pyrophosphate (P₂O₇⁴⁻), in particular Na₂H₂P₂O₇, Na₃HP₂O₇ and Na₄P₂O₇; triphosphate, polyphosphate (Mₙ₊₂PₙO₃ₙ₊₁), wherein M is an appropriate cation, e. g. pentanatriumtriphosphat (Na₅P₃O₁₀), diphosphoric acid (H₄P₂O₇), adenosine monophosphate (AMP), (C₁₀ H₁₄ N₅ O₇ P) adenosine triphosphate (ATP) (C₁₀H₁₆N₅O₁₃P₃), adenosine diphosphate (ADP) C₁₀H₁₅N₅O₁₀P₂; magnesium (ATP) (C₁₀H₁₄MgN₅O₁₃P₃), Magnesium (ADP) (C₁₀H₁₃MgN₅O₁₀P₂) and combinations of any of the foregoing;and/or wherein the at least one enzyme is selected from proteases, amylases, lipases, hydrolases, ligases, isomerases, lyases, transferases, oxidoreductases, kinases, zymases, catalases, thrombin, serine proteases, enzyme precursors such as zymogen, enzyme combinations such as bromelain.

In one embodiment, the phosphate source and/or the at least one enzyme are included in the protein source and are provided in step a) by the protein source when the protein source is provided.

In one embodiment, the phosphate source and/or the at least one enzyme are provided separately from and in addition to the protein source.

In one embodiment, in step a) said protein source, said phosphate source and said at least one enzyme are provided in a weight ratio in the range of from 100:0.1:0.001 to 100:10:5.

The present invention also relates to a composition produced by the method as defined above.

In one embodiment, the composition additionally comprises water, preferably at a weight ratio of granules of said mixture to water in a range from 1:0.1 to 1:100, wherein preferably, said granules of said mixture are dissolved, partially dissolved, emulsified or dispersed in said water. In one embodiment, said water has been added, after the granules of said composition have been formed.

In one embodiment, said composition, when administered to a patient in a suitable amount, changes the electric charge of the blood of said patient in such a manner that,
(i) when the electric charge of the blood before administration of said composition is below +5mV as initially measured value, the electric charge of the blood of said patient will increase within 8 minutes after administration of said composition, by at least 100 %, preferably at least 200 %, more preferably at least 300 %, and/or within 1 hour by at least 400 %, (ii) when the electric charge of the blood of said patient before administration of said composition is above +6 mV as initially measured value, the electric charge of the blood of said patient will decrease within 10 minutes after administration of said composition down to 1 - 20 % of the initially measured value, preferably by at least 100 % - 200 %, possibly to negative electric charge values, wherein said electrical charge of said blood is measured by a voltmeter having two electrodes and by dipping said two electrodes into a sample of blood of said patient

In the case (i) above i. e. when the electric charge of the blood before administration of said composition is below +5mV, it is also envisaged that the initial electric charge may be negative. In that case, an increase "by at least 100 %, preferably at least 200 %, more preferably at least 300 %" is meant to signify that the total increase of the electric charge of the blood will be 100 %, preferably at least 200 %, more preferably at least 300 % of the modulus of the blood electric charge. For example, if the initial blood electric charge is -2mV, an increase by at least 100 %, preferably at least 200 %, more preferably at least 300 % will lead to a blood electric charge of 0mV, +2mV, +4mV, respectively.

The present invention also relates to a composition, as defined above, for use in a method of strengthening the immune system of a patient and/or for use in a method of preventing, treating and/or alleviating a disease selected from infections, in particular viral and bacterial infections, cancer, diabetes and depression in a patient, and/or for use in a method of mood-enhancing a patient, wherein said method comprises administering a suitable amount of said composition to said patient, preferably 1 - 3 times daily.

The present invention also relates to a method of strengthening the immune system of a patient and/or of treatment of a disease and/or for mood-enhancing a patient, said method comprising administering a suitable amount of a compositing according to the present invention to a patient, wherein said disease is selected from infections, in particular viral or bacterial infections, cancer, diabetes and/or depression.

In one embodiment of said composition or of said method, said suitable amount is in the range of from 1g to 50 g, preferably 3g to 20g.

Without wishing to be bound by any theory, the inventor believes that the energy of the composition is higher, the more mechanical energy has been introduced in step b) and/or the finer the granules are that are produced by the comminuting step c). It should also be noted that the more mechanical energy is introduced in step b), the higher the temperature will rise during such step b). A person skilled in the art can modify either the mechanical energy that is introduced or the size of the granules or both in order to obtain a composition of a suitable energy. For example, a composition into which a large amount of mechanical energy has been introduced but where the granules are relatively large, for example of an average diameter of 1cm will have less energy than a composition where the same amount of mechanical energy has been introduced but additionally the granules have been produced and comminuted to such an extent that their average diameter is 50mV. A composition that has a high energy should be administered by taking appropriate precautions, ideally under the monitoring of a medical doctor. For example, blood pressure and heart rate should be monitored upon administration of a composition in accordance with the present invention. Likewise the blood electric charge should be measured upon administration of a composition in accordance with the present invention. If the blood electric charge reaches or exceeds 40mV, great care must be taken and the patient must be monitored appropriately and continuously by skilled medical staff.

The present inventors have found that in using compositions which have been prepared in accordance with the present invention, they are able to substantially alter the blood electric charge of a patient and thereby contribute to the patient's well-being and/or recovery. The inventors have noted that the blood electric charge is a measure that can be used to follow the effect of compositions in accordance with the present invention. The present inventors have noted that the blood electric charge of a patient may be different depending on its health status. Typically, the blood electric charge is measured by withdrawing a drop of blood (approximately 600-1000 µl) and measuring the voltage within that drop using two electrodes which are held within the sample. The inventors have found that such blood electric charge can be very different, depending on the health status of the respective patient/person. In a normal healthy individual, such blood electric charge is typically below +5 mV, but, after administration of an intake of a composition according to the present invention, it quickly rises by at least 100 % within 8 minutes after administration, this can be from the negative electric charge to positive. If the person/individual is ill, (s)he is likely to have a blood electric charge > +6 mV, and upon administration of a composition according to the present invention, such blood electric charge will decrease swiftly within 10 minutes after administration of said composition down to 1-20% of the initially measured value, preferably by at least 100 % - 200 %, possibly even into negative electric charge values.

This dramatic change in blood electric charge is also reflected in a patient's subjective well-being, in that the patient, after intake of a composition of the present invention will feel energetic and better within a short time. Without wishing to be bound by any theory, the present inventors believe that the compositions in accordance with the present invention have a positive effect on the energy status in the blood, as measured by the blood electric charge outlined above. It is interesting to note that typically, the blood electric charge changes much faster than the blood sugar level; an administration of a composition according to the present invention will therefore likely not have an immediate effect on the blood sugar. This will also be reflected in a patient's immune status, and a patient having been administered a composition in accordance with the present invention is likely to have an elevated immune status and be able to fight infections more efficiently.

The compositions in accordance with the present inventions also do have a blood sugar-lowering effect, albeit not as fast-acting as the effect on the blood electric charge. Typically an administration of a suitable amount of a composition according to the present invention will lead to a reduction of blood sugar down to 70-80 % of the initial value, within an hour of administration, and down to 50-60 % of the initial value, within two hours and thirty minutes of administration. Hence, this blood sugar effect is delayed and it is an effect that results in a lowering of the blood sugar.

As used herein, the term "blood electric charge" or "electric charge of the blood" is meant to refer to the voltage measured in a sample of blood of the patient, such sample comprising 600 µl - 1000 µl of blood obtained from the patient, preferably from pricking a fingertip of said patient and putting such sample on a microscope slide and measuring a voltage using a voltmeter with both electrodes inserted in said sample and held at a defined distance between the electrodes of from 3 mm to 10 mm, preferably approximately 4 mm between the electrodes. A reading is taken when the read-out values are stable, which is the case typically after 10-60 seconds, preferably approximately 30 seconds. Standards values measured for healthy patients in this manner typically lie in the range of from 1.5-4.8 millivolt.

The term "water activity (a_{w})" is meant to define the residual moisture that remains in the composition after the composition has been dried. Water activity is the quotient of the partial vapor pressure of water in a substance and the standard state partial vapor pressure of pure water at the same temperature. Water activity may take values from 0 (= completely dry) to 1 (= pure water). Compositions according to the present invention may adopt values of water activity from 0.01 to 0.99, preferably from 0.3 to 0.9, more preferably from 0.5 to 0.8. In one embodiment, compositions according to the present invention may adopt values of water activity from 0.5 to 0.7. In another embodiment, they may adopt values of water activity from 0.01 to 0.3. Water activity may be measured by any means known to a person skilled in the art, in particular resistive electrolytic hygrometers, capacitance hygrometers and dew point hygrometers. In practice, water activity is measured with an indirect method. Water activity is measured by equilibrating the liquid phase water in the composition according to the present invention with the vapor phase water in the head space of a closed chamber and measuring the relative humidity of the head space. Methods for water activity determinations are detailed for example in the OFFICIAL METHODS OF ANALYSIS OF AOAC INTERNATIONAL (1995). Different types of water activity instruments are commercially available. One uses chilled mirror dew point technology while the other utilizes relative humidity sensors that change electrical resistance or capacitance (see above).

The invention is now further described by reference to the following examples which are given to illustrate, not to limit the present invention.

### Examples

### Example 1

### Production 1 of a composition in accordance with the present invention

In a bowl cutter or mincer, 10 kg (beef roundcut), 10 g phosphate (adenosine triphosphate) and 0,5 g enzyme (tranferase) are mixed and the mixture is minced, starting from a temperature at -2 °C. The cutting, i.e. the introduction of mechanical energy, leads to a temperature increase in the mixture, and the mincing/introduction of mechanical energy takes place until a temperature of 25 °C is reached. During the cutting/mincing, no water or ice is added. Then the mincing is stopped, and the mixture being dried at a temperature of 82 °C.

The dried mixture has a water activity of approximately 0.6 and is then comminuted to a granular powder with the average diameter of the granules of said mixtures being approximately 50 µm.

The product can be stored in this state for several years and maintains its positive, health-beneficial qualities, provided that it is kept in dry conditions.

### Production 2 of a composition in accordance with the present invention

In a bowl cutter or mincer, 5 kg (tofu), 10 g phosphate (adenosine diphosphate) and 2g enzyme (cysteine protease) are mixed and the mixture is minced, starting from a temperature at +7 °C. The mincing, i.e. the introduction of mechanical energy, leads to a temperature increase in the mixture, and the mincing/introduction of mechanical energy takes place until a temperature of 21 °C is reached. During the cutting/mincing, no water or ice is added. Then the mincing is stopped, and the mixture is dried at a temperature of 78 °C.

The dried mixture has a water activity of approximately 0.6 and is then comminuted to a granular powder with the average diameter of the granules of said mixtures being approximately 20 µm.

### Production 3 of a composition in accordance with the present invention

In a bowl cutter or mincer, 10 kg (**beef roundcut**), no phosphate and no enzyme; only the meat itself is mixed and the mixture is minced, starting from a temperature at +2 °C. The cutting, i.e. the introduction of mechanical energy, leads to a temperature increase in the mixture, and the mincing/introduction of mechanical energy takes place until a temperature of 39 °C is reached. During the cutting/mincing, no water or ice is added. Then the mincing is stopped, and the mixture is dried at a temperature of 82 °C.

The dried mixture has a water activity of approximately 0.6 and is then comminuted to a granular powder with the average diameter of the granules of said mixtures being approximately 50 µm.

The product can be stored in this state for several years and maintains its positive, health-beneficial qualities, provided that it is kept in dry conditions.

### Production 4 of a composition in accordance with the present invention

In a bowl cutter or mincer, 5 kg (**milkpowder**), 20% water, 10 g phosphate (adenosine triphosphate) and 0,5 g enzyme (cysteine protease) are mixed and the mixture is minced, starting from a temperature at +18 °C. The cutting, i.e. the introduction of mechanical energy, leads to a temperature increase in the mixture, and the mincing/introduction of mechanical energy takes place until a temperature of 29 °C is reached. During the cutting/mincing, water is added. Then the mincing is stopped, and the mixture is dried at a temperature of 82 °C.

The dried mixture has a water activity of approximately 0.5 and is then comminuted to a granular powder with the average diameter of the granules of said mixtures being approximately 10 µm.

Generally speaking, with respect to the above productions 1-4, it appears that production 1 leads to a very potent composition which is capable of reaching a blood electric charge of up to 40 mV easily and swiftly. If during the production of a composition with production 1, higher temperatures, such as 35°C were allowed to be reached during step b), such resultant composition would have to be given to patients under control and with continuous monitoring by a medical doctor. The effect is less pronounced in production 2 where the blood electric charges that can be achieved thereby are lower than in production 1. Values of blood electric charge of 8 mV that can be reached by compositions produced in accordance with production 2 are typical. In production 3, the composition is capable of reaching high blood electric charges relatively easily. For example, 16 mV can be achieved without difficulties which correspond to an increase by up to 400% (if one were to start from an initial value of approximately 4 mV). In accordance with production 4, this composition led to the lowest increase in blood electric charge, reaching for example charge values of 6 mV. The efficacy of compositions produced in accordance with production 1 and 3 appears to be the highest and the longest lasting. For example, these compositions were typically capable of increasing the blood electric charge for a period of at least 24 hours, in comparison to the value measured initially.

In the examples that follow (examples 2-4), compositions according to the present invention are administered to various human patients. The human patients, prior to treatment, had consented to such treatment, but did not know the exact composition that they were administered. Moreover, they were informed about the confidentiality of such treatment and were thus bound to secrecy by a confidentiality agreement with the inventors.

### Example 2

### Effect of an embodiment of the composition according to the present invention on the electric charge of the blood of a patient

A composition according to the present invention, as produced in Example 1 (production 3), is administered to different individuals at an amount of 2 tablespoon fulls, i.e. approximately 20 g. The composition is thus orally ingested. The electric charge of the blood of the patient to whom the composition was administered is measured at various intervals after intake. The measurement is done by pricking a fingertip of a patient and allowing one drop of blood (approximately 600 µl - 1 ml) to settle on a microscope slide. The two electrodes of a standard voltmeter, such as a digital multimeter "EM393" available from Hama or a voltmeter "Voltcraft VC170-1 CAT III" available from Conrad Electronics, are dipped into the blood and held at a defined distance between the electrodes, said distance being in a range of from 3 mm to 10 mm, preferably approximately 4 mm. Readings are taken approximately 30 seconds later when the reading is stable. In a healthy patient, this is typically below +5 mV. In the present experiment of this example, the values are recorded as follows:

**Table 1: Effect of compositions according to the present invention on electric charge of the blood of patients**

| Patient | Blood electric charge | Amount (Table spoon) | time difference from intake | electric charge of the blood |
|---|---|---|---|---|
| 1. | 2.1 - 2.4 millivolt before breakfast | 2 | 4 minutes | 8.2 |
| | | | 10 minutes | 10.1 |
| | | | 30 minutes | stable at 7.2 |
| | | | 2 hours | stable at 6.2 |
| | | | after 24 hours | stable at 3.2 |
| | | | | |
| 2. | 2.6 millivolts | 2 | 10 minutes | 7.6 millivolts |
| | before breakfast | | 1 hour | 17.6 |
| | | | 2 hours + 30 minutes | stable at 6.8 |
| | | | | |
| 3. | 3.4 millivolts after lunch | 2 | 4 minutes | stable at 6.5 |
| | | | 30 minutes | stable at 7.5 |
| | | | | |
| 4. | 6.8 millivolts | 1 | 10 minutes | stable at 1.1 millivolts |
| | | | 30 minutes | stable at - 1.8 negative |

The electric charge measured for patient no. 4 was the electric charge of a patient who was sick and who was suffering from a viral infection.

It can be seen that in healthy individuals, the electric charge of the blood is below 5 mV. Within a very short time after intake, the electric charge of the blood changes dramatically such that in patients 1-3, a dramatic increase is observed even after a short time. In a sick patient, it turns out that the opposite is observed in that a substantial decrease in electric charge, possibly even into the negative range is observed.

### Example 3

### Effect of compositions according to the present invention on blood sugar

The compositions according to the present invention also have a marked effect on the blood sugar. The blood sugar is measured conventionally by Ascensia Contour from Bayer. As can be seen from the following table 2, the intake of 2 tablespoons of a composition according to the present invention (production 3) leads to a considerable decrease in blood sugar such that after 8 minutes, there are only approximately 88-95 % of the initially measured blood sugar value. After one hour, the reduction is down to 70-77 % of the initially measured value, and after two hours and thirty minutes, the reduction is down to 58 % - 60 % of the initially measured value.

**Table 2: Effect of compositions according to the present invention on the blood sugar of a patient**

| Amount of composition | time-difference after intake | Blood sugar [mg/dl] |
|---|---|---|
| Patient 1 | before intake | 330 |
| 2 x Tablespoons | 8 minutes | 299 |
| | 1 hour | 231 |
| | 2 hours + 30 minutes | 191 |
| | | |
| Patient 2 | before intake | 277 |
| 1x Tablespoon | 8 minutes | 244 |
| | 1 hour | 211 |
| | 2 hours + 30 minutes | 164 |

**Patient 3, intake product in glas of water 200ml.**

| **amount of composition** | **time-difference after intake** | **Bloodsugar [mg/dl]** | **electric charge [mV]** |
|---|---|---|---|
| **after breakfast** | | **283** | **3,2** |
| **1 table spoon** | **8 minutes** | **274** | **8,7** |
| | **1 hour** | **254** | **7,2** |
| | **2 hours + 30 minutes** | **208** | **6,2** |

blood sugar values are measured in mg/dl.

### Example 4

### Effect of compositions according to the present invention on patients with infections

The compositions according to the present invention have a remarkable effect on the well-being of patients that had previously been diagnosed with viral infections, cancer and depression.
Cancer and depression patient 1.
- diagnosis: lung cancer, depression. Weight 74kg. Surgery to take place in 3 months. Medication: Fluoxetine Hexal and Stangyl 25mg over one year only for depression.
- starting now with a daily intake of 20 grams of composition according to the present invention (production 3).
- increase of weight of 4 kg to 78 kg, patient has no depression; a positive mood and is filled with positive expectations prior to surgery;
- Operation prearrangement: detection of additional cancer in liver and kidneys.
- Patient gets depressive again;
- Patient takes composition according to the present invention again; his mood gets better, only weak signs of depression.
- 4 months later the patient plummets, goes to hospital for one week. Patient experiences weight loss and does not want to eat;

Patient becomes depressive again and dies.

Cancer and depression patient 2.
- Diagnosis: gastric and pancreatic cancer.
- Radiotherapy and multiple surgery. Weight 62kg.
- Very depressive, one year sick, depression medicine Zoloft©solution (sertraline).
- With permission from a doctor patient receives 5 grams, after 3 days 10grams and after one week 15grams of composition according to the present invention (production 3). After 3 weeks the patient gets more active, after 10 weeks gets his weight 2 kg up to 64kg. After 6 months he puts on an additional 2kg to 66kg.
- He can work from his home office half a day.
- After 12 months he works again (light physical work - cook) and manages to climb up to the 3^{rd} floor, which was not possible for him before.
- He is alive and continues to take the composition according to the present invention at 5 grams a day. When his mood goes down he takes 5 grams more.
- He is happy to work and is integrated in life.
Virus-infektion: influenza virus
Bacterial Infections: Staphylokokki, *Streptococcus pneumoniae*

Patient 1. Age 68. In the schedule monitored by a doctor, the diagnosis was a light bacterial and viral pneumonia, he was prescribed penicilin , but the patient declined and instead preferred a composition according to the present invention because he had previously experienced problems with the penicillin.

**Table 3: Effect of compositions according to the present invention**

| Patient | Sickness | amount/teaspoon (ts) x times a day | effect |
|---|---|---|---|
| age 68 male | virus infection | 2 ts x 3 | Day 1: patient feels better, has better breathing |
| | | | Day 2: gets up from bed and wants to go out (Wintertime) |
| | | | Day 3: starts to work, but still sickness symptoms |
| | | | Day 4: works with no problems, but still sickness symptoms |
| | | | Day 5: in good shape, still sick, but the sickness symptoms disappear |
| | | | Day 6: symptoms almost gone |
| | | | Day 7: symptoms gone |
| | | | |
| age 13 male | virus infection | 2 ts x 4 | Patient feels very sick, wants to stay in bed |
| | | | Day 1: no change |
| | | | Day 2: feels better and wants to get up |
| | | 2 ts x 3 | Day 3: gets sick again, feels very sick |
| | | | Day 4: gets better, starts to watch TV |
| | | | Day 5: is hungry, starts to play with computer |
| | | | Day 6: is hungry and is playing with computer 5 hours a day |
| | | | Day 7: feels good, but does not go out yet |
| | | 1 ts x 3 | Day 8: feels not good enough for school |
| | | | Day 9: is fine, feels good |
| | | 1 ts x 3 for the rest of the week | Day 10: goes to school for the rest of 3 days; no more symptoms |
| | | | Days 11-14: takes the composition prophylactically |
| | | | |
| age 51 female | virus infection | | Day 1: feels very sick |
| | | 1 ts x 3 | Day 1: takes composition 3 times |
| | | | Day 2: gets sicker, but is still in good mood. |
| | | | Day 3: gets better and feels good |
| | | 1 ts x 2 | Day 4: last day of intake, feels good. |
| age 61 female | cold | 1 ts x 3 | Patient also has an allergy. |
| | | | Day 1: cold, very sick, blocked nose, in bed. |
| | | 2 ts x 3 higher dose, can take it | Day 2: gets sicker, starts now with higher dose |
| | | | Day 3: can breathe better and feels better |
| | | | Day 4: feels sick, but starts cooking again |
| | | | Day 5: starts to work at home, does washing, cooking |
| | | | Day 6: feels very good and is happy, works the whole day |
| | | 1 ts x 2 for next 3 days | Day 7: feels good, is going out for shopping |
| | | | |
| age 46 male | cold | 2 ts x 3 | Day 1: very sick, is lying in bed, nose blocked, headache |
| | | | Day 2: feels good, but still weak |
| | | | Day 3: gets up and starts to eat and watch TV |
| | | | Day 4: goes back to work for 4 hours |
| | | | Day 5: works for 6 hours |
| | | 1 ts x 2 for 2 days | Day 6: feels good, takes it for safety reasons |

As can been seen from the table, the oral ingestion of compositions according to present invention has a marked effect on the well-being of patients diagnosed with viral infections which generally results in a shortening of sick-leave-time from work. The patients get better at a relatively early stage after ingestion of the composition according to the present invention. Their overall mood changes very quickly and they feel energetic at an early stage.

The patients express that after intake of the product, they feel a subjective improvement instantly, and they feel that their energy comes back shortly after intake. The same positive effect was also expressed by cancer patients undergoing chemotherapy who feel much stronger after intake of the composition according to the present invention.

The composition according to the present invention represent an easy to manufacture product that can be used in a variety of circumstances and helps to increase the immune status and the energy of a patient thus supporting him/her in the recovery from various diseases, including infections, cancer and depression as well as diabetes.

### Comparative Example 1

In order to compare compositions according to the present invention with commercial available meat/protein compositions, an experiment similar as described in Examples 2 and 3 is performed, wherein a beef snack commercially available, such as Jack Link's ® and a beef protein bulk powder, such as Rinderprotein Isolat 97 (Hydrobeef ^{™}) are ingested, and the blood electric charge is measured before and at time intervals after intake. Likewise, the blood sugar is measured. For the beef snack Jack Link's, the electric charge of the blood does not change at all from before ingestion to two hours after ingestion of the product. Between eight minutes after intake to one hour after intake, the blood sugar slightly increases from 208 mg/dl to 234 mg/dl. For the Beef Protein Isolate 97 (Hydrobeef ^{™}), the blood electric charge does not change at all, and the blood sugar does not change either. Hence, commercially available protein products do not have a comparable effect on the electric charge of the blood or the blood sugar, when compared with compositions according to the present invention.

**Table: Effect of commercially available protein products on electric charge of the blood an on blood sugar of patients**

| | | |
|---|---|---|
| product | Blood electric charge (mV) | Blood sugar (mg/dl) |
| Jack Link's Beef Snack (25 g) | before intake 2.3 | 208 |
| | 8 minutes after intake 1.8 | 208 |
| | 30 minutes after intake 3.1 | 233 |
| | 1 hour after intake 3.0 | 234 |
| | | |
| Beef Protein Isolate 97 | before intake 3.1 | 223 |
| (Hydrobeef ^{™}) | 8 minutes after intake 3.1 | 223 |
| (2 Tablespoons) | 30 minutes after intake 2.9 | 230 |
| | 1 hour after intake 3.3 | 219 |

The features of the present invention disclosed in the specification, and/or the claims may, both separately and in any combination thereof, be material for realizing the invention in various forms thereof.

## Claims

1. A method for producing a composition for strengthening the immune system, said method comprising:
a) providing in any order,
- a protein source,
- a phosphate source,
- at least one enzyme,
and mixing them to form a mixture;
b) introducing mechanical energy into the mixture while keeping the mixture at a temperature in the range of from -20°C to 42°C;
c) drying the mixture at a temperature in the range of from -100°C to 380°C and comminuting the mixture to granules to obtain a composition for strengthening the immune system, said composition consisting of said granules of said mixture.

2. The method according to claim 1, wherein, during step b), the introducing of mechanical energy into the mixture leads to a temperature increase of said mixture, and wherein the introducing of mechanical energy into said mixture in step b) is performed such that said temperature increase of said mixture is in a range of from 3°C to 62°C.

3. The method according to any of the foregoing claims, wherein step b) is performed by a process selected from a cutting, mixing, mincing, shaking, kneading, tumbling, rolling, pressing, beating, pushing, squeezing, rubbing, and combinations of any of the foregoing.

4. The method according to any of the foregoing claims, wherein the drying in step c) is performed
until the mixture has a water activity (a_{w}) in the range of from 0.01 to 0.99, preferably from 0.3 to 0.9, more preferably from 0.5 to 0.8.

5. The method according to any of the foregoing claims, wherein the comminuting in step c) is performed until the granules of said mixture have an average diameter in the range of from 1 µm to 10 mm, preferably 10 µm to 1 cm, more preferably 100 µm to 1 cm.

6. The method according to any of the foregoing claims, wherein the protein source is selected from meat, bones, skin, cartilage, offals, from mammals, such as cow, pig, sheep, goat, camel, horse, in particular beef, pork, lamb; birds, in particular poultry, such as chicken, duck, ostrich, goose; fish and seafood, such as lobster, shrimp, crab, crayfish; and animal products, such as milk and eggs; vegetables, in particular beans, nuts, peas, peanuts, corn, cabbage, legumes, cereals, seeds, kernels and parts and products thereof, e. g. tofu;
and/or wherein the phosphate source is selected from monosodium phosphate (NaH₂PO₄), disodium phosphate (Na₂HPO₄), trisodium phosphate (Na₃PO₄), ortho-phosphoric acid (H₃PO₄), monopotassium phosphate (KH₂PO₄), dipotassium phosphate (K₂HPO₄), tripotassium phosphate (K₃PO₄), calcium phosphate, in particular monocalcium phosphate (Ca(H₂PO₄)₂, dicalcium phosphate (CaHPO₄), tricalcium
phosphate Ca₃(PO₄)₂, magnesium phosphate, in particular monomagnesium phosphate (Mg(H₂PO₄)₂), dimagnesium phosphate (MgHPO₄), trimagnesium phosphate (Mg₃(PO₄)₂), pyrophosphate (P₂O₇⁴⁻), in particular Na₂H₂P₂O₇, Na₃HP₂O₇ and Na₄P₂O₇; triphosphate, polyphosphate (Mₙ₊₂PₙO₃ₙ₊₁), wherein M is an appropriate cation, e. g. pentanatriumtriphosphat (Na₅P₃O₁₀), diphosphoric acid (H₄P₂O₇), adenosine monophosphate (AMP)(C₁₀H₁₄N₅O₇P), adenosine triphosphate (ATP) (C₁₀H₁₆N₅O₁₃P₃), adenosine diphosphat (ADP) C₁₀H₁₅N₅O₁₀P₂; magnesium (ATP) (C₁₀H₁₄MgN₅O₁₃P₃), Magnesium (ADP) (C₁₀H₁₃MgN₅O₁₀P₂) and combinations of any of the foregoing;and/or wherein the at least one enzyme is selected from proteases, amylases, lipases, hydrolases, ligases, isomerases, lyases, transferases, oxidoreductases, kinases, zymases, catalases, thrombin, serine proteases, enzyme precursors such as zymogen, enzyme combinations such as bromelain.

7. The method according to any of the foregoing claims, wherein the phosphate source and/or the at least one enzyme are included in the protein source and are provided in step a) by the protein source when the protein source is provided.

8. The method according to any of claims 1 - 6, wherein the phosphate source and/or the at least one enzyme are provided separately from and in addition to the protein source.

9. The method according to any of the foregoing claims, wherein in step a) said protein source, said phosphate source and said at least one enzyme are provided in a weight ratio in the range of from 100:0.1:0.001 to 100:10:5.

10. A composition produced by the method according to any of claims 1 - 9.

11. The composition of claim 10, additionally comprising water, preferably at a weight ratio of granules of said mixture to water in a range from 1:0.1 to 1:100, wherein
preferably, said granules of said mixture are dissolved, partially dissolved, emulsified or dispersed in said water.

12. The composition according to any of claims 10 - 11, wherein said composition, when administered to a patient in a suitable amount, changes the electric charge of the blood of said patient in such a manner that,
(i) when the electric charge of the blood before administration of said composition is below +5mV as initially measured value, the electric charge of the blood of said patient will increase within 8 minutes after administration of said composition, by at least 100 %, preferably at least 200 %, more preferably at least 300 %, and/or within 1 hour by at least 400 %,
(ii) when the electric charge of the blood of said patient before administration of said composition is above +6 mV as initially measured value, the electric charge of the blood of said patient will decrease within 10 minutes after administration of said composition down to 1 - 20 % of the initially measured value, preferably by at least 100 % - 200% %, possibly to negative electric charge values, wherein said electrical charge of said blood is measured by a voltmeter having two electrodes and by dipping said two electrodes into a sample of blood of said patient

13. The composition according to any of claims 10 - 12, for use in a method of strengthening the immune system of a patient and/or for use in a method of preventing, treating and/or alleviating a disease selected from infections, in particular viral and bacterial infections, cancer, diabetes and depression in a patient, and/or for use in a method of mood-enhancing a patient, wherein said method comprises administering a suitable amount of said composition to said patient, preferably 1 - 3 times daily.

14. A method of strengthening the immune system of a patient and/or of treatment of a disease and/or for mood-enhancing a patient, said method comprising administering a suitable amount of a compositing according to any of claims 10 - 12 to a patient, wherein said disease is selected from infections, in particular viral or bacterial infections, cancer, diabetes and/or depression.

15. The composition according to any of claims 12 and 13 or the method according to claim 14, wherein said suitable amount is in the range of from 1g to 50 g, preferably 3g to 20g.
